# EUROPEAN PATENT APPLICATION

(11) **EP 4 212 153 A1**
(43) Date of publication of application: **19.07.2023**
(21) Application number: 21865517.3
(22) Date of filing: 02.04.2021
(51) Int. Cl.: A61K 31/277, A61K 31/4406, A61K 9/06, A61P 27/02, A61P 5/26

(54) **USE OF ESTER GROUP-CONTAINING AROMATIC PROPIONAMIDE COMPOUND IN PREPARATION OF MEDICINE FOR TREATING DRY EYE SYNDROME**

(30) Priority: 14.09.2020 CN 202010959998
(71) Applicant: Changchun Genescience Pharmaceutical Co., Ltd., Changchun, Jilin 130012 (CN)
(72) Inventor: ZHU, Xinfa, Zhejiang 315102 (CN); ZHU, Ran, Zhejiang 315102 (CN)
(74) Representative: Gille Hrabal Partnerschaftsgesellschaft mbB Patentanwälte
(86) International application number: PCT/CN2021/085266
(87) International publication number: WO 2022/052454

(57) **Abstract**

The use of an ester group-containing aromatic propionamide compound in the preparation of a medicine for treating dry eye syndrome.

## Description

### TECHNICAL FIELD

The present disclosure belongs to the technical field of medicines, and particularly relates to use of an ester group-containing aromatic propionamide compound for the manufacturing of a medicament for the treatment of dry eye syndrome.

### BACKGROUND

Dry eye syndrome refers to a general term for a variety of diseases characterized by decreased tear film stability resulting from abnormal tear quality or quantity or abnormal kinetics for any reason, accompanied by ocular discomfort and/or ocular surface tissue lesions. It may cause lesions of cornea and conjunctiva and affect eyesight. The morbidity of dry eye syndrome is greatly increased due to the continuously increased eye use time of people with the wide use of computers and smart phones in recent years, as well as the influence of bad eye use habits. Dry eye syndrome has become a common disease in the world. In our country, the morbidity of dry eye syndrome is gradually increasing, and this disease tends to develop in younger populations. Research shows that the inflammatory reaction of ocular surface tissues is the main cause of the reduction of tear secretion in dry eye syndrome, and the existing treatment of dry eye syndrome mainly includes artificial tears, corticosteroid hormone, cyclosporine A and other immunosuppressive agents. However, the artificial tear is only a substitute for tear and has no treatment effect; the drugs such as corticosteroid hormone and cyclosporine A have certain toxic and side effects on the ocular surface after long-term use. Therefore, a drug with good effect and no obvious local and systemic toxic and side effects after long-term use is urgently needed in clinic.

### SUMMARY

The object of the present disclosure is to provide use of an ester group-containing aromatic propionamide compound for the manufacturing of a medicament for the treatment of dry eye syndrome.

To achieve the object above, the following technical solution is adopted in the present disclosure:

Provided is use of an ester group-containing aromatic propionamide compound for the manufacturing of a medicament for the treatment of dry eye syndrome.

Preferably, the dry eye syndrome is one caused by sex hormone imbalance.

Preferably, the ester group-containing aromatic propionamide compound is used in combination with a pharmaceutically acceptable excipient for the manufacturing of the medicament for the treatment of dry eye syndrome.

Preferably, the ester group-containing aromatic propionamide compound and the pharmaceutically acceptable excipient are manufactured into a salve.

Preferably, the pharmaceutically acceptable excipient is mineral oil and vaseline.

Preferably, the ester group-containing aromatic propionamide compound is C₂₂H₁₉F₃N₄O₄ or C₂₂H₁₈F₄N₄O₄, wherein reference can be made to the content of the patent document with the application No. 2014103602169 for the preparation method and the structural formula of C₂₂H₁₉F₃N₄O₄ or C₂₂H₁₈F₄N₄O₄.

Preferably, an effective dose of the C₂₅H₁₇F₃N₄O₄ is 0.5-6.75 mg/kg.

The present disclosure has the following advantages: the ester group-containing aromatic propionamide compound provided herein is a novel selective nonsteroidal androgen receptor regulator and can regulate androgen receptor, and thus it can be used for the treatment of dry eye syndrome caused by androgen imbalance; besides, animal experiments show that this compound can increase the tear secretion of castrated rabbits, reduce the corneal fluorescence staining score and increase the tear film break-up time, and has marked ameliorating effect on the experimental dry eye syndrome.

### BRIEF DESCRIPTION OF THE DRAWINGS

FIG. 1 is a schematic diagram showing the examination results of the harderian gland pathological sections of each group.
FIG. 2 is a schematic diagram showing the examination results of the meibomian gland pathological sections of each group.
FIG. 3 is a graph showing the effect on the tear film break-up time of male cynomolgus monkey models.
FIG. 4 is a column chart showing the effect on the tear film break-up time of male cynomolgus monkey models.
FIG. 5 is a schematic diagram showing the corneal fluorescence staining results of eyes in the male cynomolgus monkey experiment.
FIG. 6 is a graph showing the effect on the tear secretion of modeled animals in the male cynomolgus monkey experiment.
FIG. 7 is a column chart showing the effect on the tear secretion of modeled animals in the male cynomolgus monkey experiment.
FIG. 8 is a schematic diagram showing the change in body weight of male New Zealand white rabbits in each group.
FIG. 9 shows the effect on the tear film break-up time of male New Zealand white rabbits in each group.
FIG. 10 is a schematic diagram showing the fluorescence staining results of male New Zealand white rabbits in each group.
FIG. 11 is a schematic diagram showing the effect on the tear secretion of male New Zealand white rabbits in each group.

### DETAILED DESCRIPTION

The following experiment was conducted to confirm the effect of the ester group-containing aromatic propionamide compound of the present disclosure on the treatment of dry eye syndrome. The EG compounds mentioned herein are all ester group-containing aromatic propionamide compounds, and EG-12, EG-312, EG-17 and EG-317 are C₂₂H₁₉F₃N₄O₄, C₂₂H₁₈F₄N₄O₄, C₂₅H₁₇F₃N₄O₄ and C₂₅H₁₆F₄N₄O₄, respectively, wherein reference can be made to the content of the patent document with the application No. 2014103602169 for their specific preparation methods and structural formulas.

### 1 Name and objective of experiment

### 1.1 Name of experiment

Ameliorating effect of EG compounds on dry eye syndrome caused by castration of male rabbits

### 1.2 Objective of experiment

To observe the ameliorating effect of test substances EG-12, EG-312, EG-17 and EG-317 on dry eye syndrome caused by castration of male rabbits

### 2 Start and end date of experiment: Aug. 5, 2014-Oct. 27, 2014

### 3 Names, stability and other characteristics of test substances

### 3.1 Names of test substances: EG-12 (C₂₂H₁₉F₃N₄O₄), EG-312 (C₂₂H₁₈F₄N₄O₄), EG-17 (C₂₅H₁₇F₃N₄O₄) and EG-317 (C₂₅H₁₆F₄N₄O₄)

Storage conditions of test substances: sealed, stored at room temperature in shade

### 3.2 Preparation of test substances

Selection of vehicles for test substances: mineral oil and vaseline

Preparation method of test substances: grinding, adding mineral oil and vaseline, and dissolving to obtain paste

Frequency of preparation of test substances: one preparation for three days

### 4 Species, number, sex, weight range, source, animal certificate No. and issuing institute, and feeding conditions of experimental animals

### 4.1 Species: Japanese big-ear white rabbit

Grade: normal
Number and sex of animals predetermined to purchase: 45, male
Number and sex of animals predetermined to use: 45, male
Weight: an average weight of 2.4-2.8 kg at the beginning of the experiment
Source: Zhengzhou Kangda Biology Company.
Production license No. of experimental animals: SCXK (Yu) 2011-0001
Animal certification No.: 41001900000233

### 4.2 Feeding conditions

Feeding room: normal environment
Temperature: 20-24 °C, daily temperature difference: 0-4 °C
Relative humidity: 40-66%

### 5 Type, source, lot number and quality of animal feed, drinking water and bedding

### 5.1 feed

Name: rabbit feed
Manufacturer: supplied by Henan Experimental Animals Center
Address: No. 40, University Road, Zhengzhou
License No.: SCXK (Yu) 2005-0001
Sterilization method: Co⁶⁰ irradiation
Feeding method: sufficient supply and free choice feeding
Reference standard: GB 14924.3-2001
Examination of feed: the feed meets the nutrition and sanitation standards, and the examination report was provided by Supervision, Inspection and Testing Center of Agricultural Products Quality, Ministry of Agriculture (Zhengzhou), Henan Provincial Center for Disease Control and Prevention, and Henan Quality Supervision and Test Station of Laboratory Animals.

### 5.2 Drinking water

Type: sterile water
Sterilization method: automatic flushing of reverse osmosis system and on-line circulating disinfection
Water supply by using LAWS-40T drinking water processor for experimental animals
Water supply method: filled in a drinking water bottle for free intake.
Examination of water quality: the water meets the sanitation standard, and the detection report was provided by National City Water Supply Water Quality Monitoring Network, Zhengzhou Monitoring Station.

### 5.3 Cage and management

Cage: RS-12 stainless steel flush-type laboratory cage for rabbits (2000×650×1720 mm³).
Number of animals per cage: 1 rabbit/cage.

### 6 Animal quarantine

7 days of quarantine, general condition observed every day in the quarantine period. Body weight was measured on the first day and the last day of the quarantine period.

### 7 Grouping and identifying method for experimental animals

### 7.1 Identifying method for animals

7.1.1 Quarantine period: fed in separate cages, tag hung on each cage, and no marking of animals.
7.1.2 Administration observation period: marked by staining with saturated picric acid solution, and 5 rabbits in each group were marked in the following ways, respectively: head, back, tail, head and back, and white.
7.2 Grouping of experimental animals: after the quarantine period was finished, 5 rabbits were randomly selected according to the body weight and used in the normal control group, and the rest rabbits were used in the modeling group. After successful modeling was confirmed by detection, the modeled rabbits were randomly divided into 8 groups according to the body weight, namely a model group, a solvent control group, a positive drug 1 (carbomer eye drop) group, a positive drug 2 (testosterone propionate injection) group and test substance groups.

### 8 Source of control substance and positive drugs

8.1 0.9% normal saline (manufacturer: Henan Taloph Pharmaceutical Stock Co., Ltd., lot#: 13062552); carbomer eye drops (manufacturer: Dr.GerhardMann, Chem.-Pharm. Fabrik GmbH, lot#: 9113); testosterone propionate injection (manufacturer: Shanghai CP General Pharmaceutical Co., Ltd., lot#: 130403); mineral oil (manufacturer: Sigma, lot#: M5904); vaseline (manufacturer: Nanchang Baiyun Pharmaceutical Co., Ltd., lot#:140501); pentobarbital sodium (manufacturer: Sigma, lot#: P3761); fluorescein sodium (Guangzhou Baiyunshan Mingxing Pharmaceutical Co., Ltd., lot#: 130926).

### 9 Route of administration, dose and frequency of test substances and control substance

9.1 Administration method: topical administration to the eyes for all the groups.
9.2 Quarantine period: Aug. 5, 2014-Aug. 11, 2014
9.3 Date of grouping: Aug. 12, 2014, Sept. 15, 2014
9.4 Date of modeling: Aug. 12, 2014
9.5 Administration time: Sept. 15, 2014
9.6 Animal sacrifice time: Oct. 27, 2014
9.2 Duration of administration: two consecutive weeks from Sept. 15, 2014 to Sept. 28, 2014.
9.3 Administration frequency: twice daily (once in the morning and once in the afternoon) for the positive 1 group (carbomer eye drops), and once daily (at specific time in the morning) for the remaining groups.

### 9.4 Administration dose

Normal control group: 0.9% normal saline, 2 drops per eye for each time.

Model control group: 0.9% normal saline, 2 drops per eye for each time.

Solvent control group: (1 mL of mineral oil + 0.5 g of vaseline) for three days, about 0.05 g/eye, 0.1 g/rabbit.

Positive drug 1 group (carbomer eye drops): carbomer eye drops, 1 drop per eye for each time.

Positive drug 2 group (testosterone propionate injection): testosterone propionate injection with the specification of 25 mg/1 mL; calculated based on 20 drops for 1 mL to get 1.25 mg/drop; 2 drops per eye for each time, 5.0 mg/rabbit and 2.5 mg/eye.

Test substance groups (EG-N): (150 mg + 1 mL of mineral oil + 0.5 g of vaseline) for three days, 5 rabbits × 3 days = 15 times, i.e., 10 mg/rabbit, 5 mg/eye.

The body weight was measured once per week.

### 10 Instruments used

Electronic balance (Shanghai Precision Instrument Co., Ltd., JA1003N); scale (Shanghai Yousheng Weighing Apparatus Co., Ltd., ASC-A); slit-lamp microscope (Jiangxi Fenglin Optical Instrument Co., Ltd., LYL-1).

### 11 Experimental method and detection indexes

11.1 Modeling method: the rabbits for operation were each subjected to general anesthesia by injection of pentobarbital sodium (50 mg.kg⁻¹) into ear vein, and sterilized conventionally. An aseptic towel was spread out, testicles at two sides were sequentially squeezed into the scrotum from abdominal cavity and clenched to prevent them from sliding, a small opening was cut on the scrotum with a sterilized blade, the testicles were squeezed out by force, the spermatic vein and vas deferens were ligated, the testicles and epididymis were cut out, the scrotum skin was sutured, and iodine tincture was coated locally to prevent infection.
11.2 Administration method: administration was started on day 35 after the operation, at a prescribed dose for 14 consecutive days.
11.3 Detection indexes: for each group, basal tear secretion (Schirmer I test), corneal fluorescence staining (PI) and tear film break-up time (BUT) were detected before the modeling and on days 0, 7, and 14 after the administration by performing surface anesthesia.
11.3.1 Basal tear secretion (Schirmer I test): the 5 mm forepart of a qualitative filter paper (35 mm×6 mm) was reversely folded and placed in the lower eyelid bitamporal conjunctival sac, and the tear infiltration length (including the reversely-folded segment) of the test paper after 5 min was detected and recorded.
11.3.2 Scoring criteria for corneal fluorescence staining (PI): 1% fluorescein sodium was applied to both eyes of a rabbit without subsequent rinsing, and a slit-lamp microscope was used for observation. Scoring 0 for no staining at corneal epithelium; scoring 1 for staining area of less than 25%; scoring 2 for staining area of 25%-50%; scoring 3 for staining area of 50%-75%; scoring 4 for staining area of greater than 75%.
11.3.3 Tear film break-up time (BUT): a glass rod was dipped in fluorescein to drip it into the lower conjunctival sac of the rabbit, and after the blinking, the time from opening eyes after the last blinking to the appearance of the first black spot at the cornea was recorded under a slit lamp.
11.4 Histopathology examination: left harderian gland and eyelid specimens were taken, fixed with 10% formaldehyde solution, dehydrated with ethanol, embedded with paraffin, sectioned and stained with conventional HE, and then the harderian gland and meibomian gland were examined under an optical microscope.

### 12 Method for data statistics and processing

Data were expressed as **x̅** ± s and analyzed by one-way ANOVA, LSD was used to compare data between two groups, and the test level α = 0.05.

### 13 Experimental results

13.1 General state and body weight of rabbits in each group: within 5 days after modeling, the rabbits in the modeling group showed reduced exercise frequency, poor hair color and relatively watery feces; during the administration period, the rabbits in each group showed good general state in terms of appearance, movement, hair, feces and the like, and no marked toxicity symptoms related to the drug were observed. Due to the influence of castration operation, the body weight of the rabbits in each group was lower than that of the normal control group, it tended to increase along with the experiment. The results are shown in Table 1.

**Table 1: Weight change of rabbits in each group (x̅ ±s, Kg)**

| Group | n | Before operation | After operation | Day 7 of administration | Day 14 of administration | Day 7 after drug withdrawal | Day 14 after drug withdrawal | Day 21 after drug withdrawal | Day 28 after drug withdrawal |
|---|---|---|---|---|---|---|---|---|---|
| Normal control group | 5 | 2.4±0.15 | 2.5±0.42 | 2.8±1.87 | 2.9±2.00 | 3.1±1.47 | 3.2±1.67 | 3.5±1.08 | 3.9±1.44 |
| Modeling group | 45 | 2.5±0.33 | 2.2±0.84 | - | - | - | - | - | - |
| Model control group | 5 | - | - | 2.4±1.01 | 2.8±1.35 | 3.1±1.22 | 3.3±1.72 | 3.3±2.54 | 4.0±1.75 |
| Solvent control group | 5 | - | - | 2.3±1.21^{∗} | 2.5±1.04 | 2.8±1.22 | 3.2±1.75 | 3.6±1.46 | 3.8±1.40 |
| Positive 1 group | 5 | - | - | 2.3±0.97^{∗} | 2.6±1.11 | 2.9±1.41 | 3.0±1.55 | 3.3±1.72 | 3.7±1.65 |
| Positive 2 group | 5 | - | - | 2.4±1.10 | 2.6±1.05 | 3.0±1.21 | 3.3±1.31 | 3.6±1.07 | 4.0±1.20 |
| EG-12 | 5 | - | - | 2.0±2.45^{∗} | 2.3±1.77^{∗} | 2.8±1.56 | 3.3±1.19 | 3.4±1.81 | 3.9±1.70 |
| EG-312 | 5 | - | - | 2.3±1.14^{∗} | 2.5±1.17 | 2.7±1.42 | 3.0±1.52 | 3.4±1.31 | 3.6±1.43 |
| EG-17 | 5 | - | - | 2.4±1.24* | 2.9±1.48 | 3.2±1.24 | 3.5±1.45 | 3.9±1.66 | 4.1±1.58 |
| EG-317 | 5 | - | - | 2.4±1.73 | 2.6±2.16 | 3.0±1.87 | 3.3±1.94 | 3.7±1.54 | 4.0±1.75 |

| | | | | | | | | | |
|---|---|---|---|---|---|---|---|---|---|
| * **P** < **0.05, compared with the normal control group** | | | | | | | | | |

13.2 Compared with the normal control group, the tear secretion of the rabbits in the model control group was reduced markedly, and the difference was statistically significant (P < 0.05); the corneal fluorescence staining score was significantly increased, and the difference was statistically significant (P < 0.05); the tear film break-up time was significantly reduced, and the difference was statistically significant (P < 0.05). This suggested that the modeling was successful. (2) Compared with the model group and the solvent group, the tear secretion of the positive drug 1 (carbomer eye drops) group, the positive drug 2 (testosterone propionate injection) group, the EG-12 group and the EG-312 group was increased markedly, and the difference was statistically significant (P < 0.05); the results are shown in Table 2.

**Table 2: Comparison of tear secretion at different time points for each group (x̅ ±s, mm)**

| Group | n | Before operation | Before administration | Day 7 of administration | Day 14 of administration | Day 28 after drug withdrawal |
|---|---|---|---|---|---|---|
| Normal control group | 5 | 18.7±2.17 | 19.4±2.08 | 19.2±2.64 | 20.6±3.41 | 19.7±2.54 |
| Modeling group | 45 | 18.9±4.28 | 10.1±3.01^{∗} | - | - | - |
| Model control group | 5 | - | - | 9.5±2.04^{∗} | 9.4±2.15* | 9.2±2.44^{∗} |
| Solvent control group | 5 | - | - | 9.8±1.42^{∗} | 9.8±3.44^{∗} | 9.5±1.72^{∗} |
| Positive 1 group | 5 | - | - | 16.5±2.18^{△▲} | 16.7±3.20^{△▲} | 12.1"=2.53^{∗△▲} |
| Positive 2 group | 5 | - | - | 12.7±2.84^{∗} | 14.8±3.17^{∗△▲} | 12.2±3.48^{∗} |
| EG-12 | 5 | - | - | 13.2±3.13^{∗} | 15.4±2.07^{∗△▲} | 12.0±2.62^{∗} |
| EG-312 | 5 | - | - | 13.4±3.92* | 15.2"=2.67^{∗△▲} | 10.1±1.95^{∗} |
| EG-17 | 5 | - | - | 10.5±2.53^{∗} | 12.4±2.11* | 9.2±1.38^{∗} |
| EG-317 | 5 | - | - | 10.2±1.71^{∗} | 11.7±4.08^{∗} | 10.2±2.86^{∗} |

| | | | | | | |
|---|---|---|---|---|---|---|
| * **P < 0.05, compared with the normal control group; ^{Δ} P < 0.05, compared with the model control group;** ^{▲} **p < 0.05, compared with the solvent control group** | | | | | | |

13.3 Comparison of corneal fluorescence staining scores at different time points for each group: the corneal staining performed on day 35 after operation showed that the modeling group was markedly increased compared with the normal control group, and P < 0.05, indicating that the modeling was successful; on day 7 of administration, the rabbits in the two positive groups, the EG-12 group and the EG-312 group tended to reduce in terms of corneal and conjunctival staining compared with the model group and the solvent control group, but the difference was not significant, and the rabbits in the EG-17 group and the EG-317 group showed no difference in terms of corneal and conjunctival staining compared with the model group and the solvent control group; on day 14 of administration, the rabbits in the two positive groups, the EG-12 group and the EG-312 group showed significant reduction in terms of corneal and conjunctival staining score compared with the model group and the solvent control group, and the difference was statistically significant, and the rabbits in the EG-17 group and the EG-317 group showed no significant difference in terms of corneal staining compared with the model group and the solvent control group; on day 28 after drug withdrawal, the rabbits in each administration group showed increased conjunctival staining score, which was significantly different from that of the normal control group, wherein P < 0.05; the results are shown in Table 3.

**Table 3. Comparison of corneal staining scores at different time points for each group (x̅ ±s)**

| Group | n | Before operation | Before administration | Day 7 of administration | Day 14 of administration | Day 28 after drug withdrawal |
|---|---|---|---|---|---|---|
| Normal control group | 5 | 0±0 | 0±0 | 0±0 | 0±0 | 0±0 |
| Modeling group | 45 | 0±0 | 3.3±1.14^{∗} | - | - | - |
| Model control group | 5 | - | - | 3.4±1.01^{∗} | 3.6±0.82^{∗} | 3.6±1.15^{∗} |
| Solvent control group | 5 | - | - | 3.2±1.17^{∗} | 3.2±1.25^{∗} | 3.4±1.31^{∗} |
| Positive 1 group | 5 | - | - | 2.8±0.62^{∗} | 1.8±0.36^{∗△▲} | 2.8±1.17^{∗} |
| Positive 2 group | 5 | - | - | 2.8±0.22^{∗} | 2.2±0.57^{∗} | 3.0±1.22^{∗} |
| EG-12 | 5 | - | - | 2.6±1.44^{∗} | 2.2±11^{∗△▲} | 2.6±1.44^{∗△▲} |
| EG-312 | 5 | - | - | 2.8±1.23^{∗} | 2.4±0.88^{∗△▲} | 3.0±1.84^{∗} |
| EG-17 | 5 | - | - | 3.2±1.69^{∗} | 3.2±2.04^{∗} | 3.2±1.61^{∗} |
| EG-317 | 5 | - | - | 3.4±1.09^{∗} | 3.2±1.56^{∗} | 3.4±1.48^{∗} |

| | | | | | | |
|---|---|---|---|---|---|---|
| **P** < **0.05, compared with the normal control group; ^{Δ} P < 0.05, compared with the model control group; ^{▲} p < 0.05, compared with the solvent control group** | | | | | | |

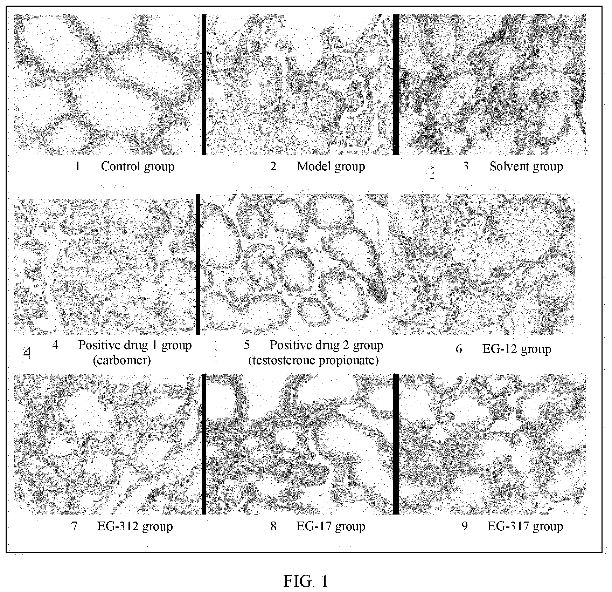

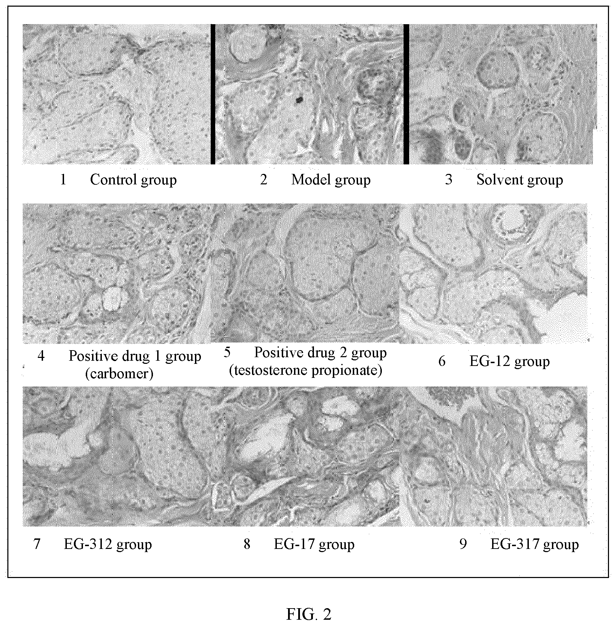

13.4 The results of histopathological examination are shown in FIGs. 1-2.

**Control group:** the harderian gland and meibomian gland tissues were arranged in order, and cellular degeneration necrosis, fibrous tissue hyperplasia and inflammatory cell infiltration were not found.

**Model group and solvent group:** harderian gland: the tissue arrangement was disordered, and degeneration necrosis of a large number of acinar cells was observed, fibrous tissues of necrotic foci were proliferated, and local inflammatory cell infiltration was found. Meibomian gland: the wall of the duct became thin, the lumen was enlarged, and fibrous connective tissue was increased and fibrosis was present. Part of the acinus didn't have normal acinus structure and showed disordered tissue structure. The cell volume was reduced, the cytoplasm was markedly reduced, cell nucleus were densely crowded and the acinus was in a atrophied state.

**Positive drugs and therapeutic agents:** the EG-12 group and the positive 2 group (testosterone propionate) showed marked ameliorating compared with the model group. The harderian gland epithelia were regularly arranged, part of the acinar epithelial cells were slightly degenerated, the fibrous connective tissue was slightly proliferated, and no marked inflammatory cell infiltration was found. The atrophy of meibomian gland epithelium was reduced markedly, the cell volume tended to be normal, the nuclear-cytoplasmic ratio was moderate, and the fibrous connective tissue was not markedly proliferated. The harderian gland and meibomian gland of other groups all showed improvement of different degrees compared with the model group, the acinar tissue cells were arranged more tidily compared with the model group, the number of degenerated and necrotic cells was reduced, and the local inflammatory cell infiltration and fibrous tissue hyperplasia were reduced to different degrees. The atrophy degree of meibomian gland epithelial cells was reduced markedly, and the connective tissues around the acini and duct were moderately proliferated.

Conclusion: the EG-12 and EG-312 can increase tear secretion of castrated rabbits, reduce corneal fluorescence staining score and increase tear film break-up time and have marked ameliorating effect on experimental dry eye syndrome. The EG-17 and EG-317 have no marked effect on experimental dry eye syndrome.

The pharmacodynamics of the tested compound EG017 for dry eye syndrome of male cynomolgus monkeys caused by castration is further studied below to show its effect.

### Experimental materials

### Experimental drug and reagents

EG017: lot#: Q18-045; supplier: 2Y-Chem, Ltd.; physical properties: off-white powder; storage conditions: sealed and stored at room temperature.

Sodium carboxymethylcellulose (CMC-Na): lot#: C1814029; manufacturer: Aladdin; physical properties: off-white powder; storage conditions: stored in dry environment and at room temperature.

Fluorescein sodium: lot#: BCBQ5572V; manufacturer, Sigma-Aldrich (Shanghai) Trading Co., Ltd.; physical properties: orange-red powder; storage conditions: sealed and stored in dry environment.

Zoletil 50 (Virbac-50); lot#: 6VUL; manufacturer, Virbac S.A., France; storage conditions: sealed and stored at room temperature.

| **Instruments** | **Model** | **Manufacturer** |
|---|---|---|
| Slit-lamp microscope | KOWA PRODUCTSL-17 | KOWA |
| Precision electronic balance | EX224ZH, B701542313 (numbering) | Ohaus Instrument (Changzhou) Co., Ltd. |
| Electronic scale | TCS-21 | GSS Scale (Suzhou) Co., Ltd. |
| High-speed refrigerated centrifuge | ST 16R | ThermoFisher |

### Experimental animals

42 male cynomolgus monkeys (Suzhou Xishan Zhongke Laboratory Animal Co., Ltd.), normal grade, weighed 4.0-9.0 kg, animal certification No.: 201817192. All the cynomolgus monkeys were fed in an environment featuring 12-hour light/dark cycle, temperature controlled at a constant temperature (22+4 °C) and 40%-70% humidity, air change was performed 10 to 20 times per hour in the feeding room, the animals were fed with normal feed, and the body weight was recorded once per week.

### Experimental method

### Experimental period

### Start date of in vivo experiment: Aug. 27, 2018; end date of in vivo experiment: Dec. 11, 2018

### Experimental procedures

Grouping: all animals were randomly divided into 6 groups based on the body weight, and the basal tear secretion and tear film break-up time before modeling, namely the non-model solvent control group (sham), the model group (Model), the EG017 low dose 0.5 mg/kg group, the EG017 medium dose 1.5 mg/kg group, the EG017 high dose (H) 4.5 mg/kg group, 7 animals per group.

Castration operation for modeling: after one-week adaptation period/baseline data acquisition, the animals were each subjected to two-sides castration operation: the monkeys were fasted and then subjected to general anesthesia by intramuscular injection of Zoletil 50 (5 mg/kg) and sterilized conventionally. An aseptic towel was spread out, testicles at two sides were then squeezed into the scrotum from abdominal cavity and clenched, a small opening was cut on the scrotum with a sterilized blade, the testicles were then squeezed out by force, the spermatic vein and vas deferens were ligated, the bilateral testicles and epididymis were cut out, the scrotum skin was sutured, and iodine tincture was coated locally. After the operation, injection of antibiotics was performed for one week to prevent infection.

End-point acute modeling by cold air stimulation: given that the modeling of the cynomolgus monkeys by castration operation took a relatively long time, in order to enhance the model effect, before the indexes of animals in each group (except for the sham group) were detected for the last time, the upper eyelid and the lower eyelid of the animal were fixed to expose the eyeballs, and a high-power electric blower (cold air mode) was used to blow at the eyes of the animal from a position 10 cm away from the eyes to contiguously stimulate the eyes of the animal for 3 minutes.

Administration: animals were given the corresponding therapeutic drug at the corresponding time according to Table 4. The non-model solvent control (Sham) group and the Model group were subjected to intragastric administration of 0.5% CMC-Na solvent, and the low dose, medium dose and high dose test drug groups were subjected to intragastric administration of EG017 solutions (0.5 mg/kg, 1.5 mg/kg and 4.5 mg/kg, respectively). All groups were subjected to administration for 14 consecutive weeks at a volume of 5 mL/kg once daily. The body weight of animals was measured once a week and clinical observation was performed twice daily.

**Table 4: Administration for each group**

| **Group** | **Number** | **Operation model** | **Test drug** | **Administration dose (mg/kg)** | **Administration volume (mL/kg)** | **Route of administration** | **Frequency and period of administration** |
|---|---|---|---|---|---|---|---|
| Sham | 7 | Sham | 0.5%CMC-Na | 0 | 5 | Oral administration | Once daily, 14 weeks |
| Model | 7 | Castration | 0.5%CMC-Na | 0 | 5 | Oral administration | Once daily, 14 weeks |
| EG017 low dose | 7 | Castration | EG017 | 0.5 | 5 | Oral administration | Once daily, 14 weeks |
| EG017 medium dose | 7 | Castration | EG017 | 1.5 | 5 | Oral administration | Once daily, 14 weeks |
| EG017 high dose | 7 | Castration | EG017 | 4.5 | 5 | Oral administration | Once daily, 14 weeks |

Ophthalmic examination: animals were fasted and then subjected to the following ophthalmic examination after general anesthesia induced by intramuscular injection of Zoletil 50 (5 mg/kg).

The detection items were tear film break-up time, corneal fluorescence staining and basal tear secretion, successively.
a. Tear film break-up time (BUT): the tear film break-up time was detected once before modeling/administration, and was detected once every 2 weeks after administration. For each detection, each of the left and right eyes was subjected to measurement 3 times, and an average value was obtained.
   Measuring method: 50 µL of 0.25% fluorescein sodium was dripped into each eye of a monkey without subsequent washing, and after blinking for 2-3 times, the time from opening eyes after the last blinking to the appearance of the first black spot at the cornea was observed with a slit lamp under a diamond blue optical filter.
b. Corneal fluorescence staining score: the corneal fluorescence staining score was measured once before modeling/administration, and was evaluated once every 2 weeks after administration, once for each of the left and right eyes.

Evaluation method: after the tear film break-up time measurement was completed, excess fluorescein solution was removed by rinsing with normal saline, and observation was performed with a slit lamp under a cobalt blue optical filter.

A 12-scoring method was used for fluorescein staining scoring: the cornea was divided into 4 quadrants, each quadrant scoring 0-3 (scoring 0 for no staining, scoring 1 for 1-30 staining spots, scoring 2 for staining spots of greater than 30 and no fusion of staining, and scoring 3 for fusion of staining spots, filaments and ulcers at the cornea). c. Tear secretion (Schirmer I test): the tear secretion was detected once before modeling/administration, and measured once every two weeks after administration, once for each of the left and right eyes.

Measuring method: after the corneal fluorescence staining score measurement was completed, excess saline flushing fluid was gently wiped away (no contact with the eyeball), and an interval (about 5 minutes) was then provided to eliminate the effect of corneal fluorescence staining on tear secretion: the forepart of a commercial test paper for tear detection was reversely folded and placed in the lower eyelid bitamporal conjunctival sac, and the tear infiltration length of the test paper after 3 min was detected and recorded.

### Preparation of formulations

0.25% fluorescein sodium: fluorescein sodium powder was precisely weighed out and dissolved in 0.9% sodium chloride injection, thus preparing the 0.25% fluorescein sodium solution.

Preparation of 0.5% CMC-Na vehicle: a sufficient amount of CMC-Na powder was weighed out and dissolved in double distillation water to prepare a CMC-Na clear solution with a final concentration of 0.5% for later use.

Preparation of EGO17 solution: according to the body weight of animals in each group, a proper amount of EG017 powder was weighed out one day before administration and placed in a mortar; a small amount of vehicle was added, and the mixture was fully ground; the drug liquid obtained by grinding was transferred into a graduated vessel, and the mortar was repeatedly washed with a proper amount of vehicle until no drug powder adhered to the mortar; a proper amount of vehicle was added into the graduated vessel containing the drug liquid until the administration concentration was reached, and the mixture was continuously stirred by using a magnetic stirrer until the drug liquid was completely mixed to form a suspension; the suspension was stored at 2-8 °C overnight, and administered in the morning of the next day; the drug liquid was transferred to an environment at room temperature at least 30 minutes earlier and continuously stirred with a magnetic stirrer until the administration was completed.

### Data processing

All data were expressed as Mean±SEM. For the experimental data, the one-way ANOVA and the 2-tailed student's t-test were used to analyze and compare the data of the groups.

### Experimental results

In this experiment, according to the progress and data of the experiment, the original experiment scheme was adjusted and revised a plurality of times, including (1) the treatment period was prolonged by 6 weeks, and the experiment operation and the operation frequency in the prolonged period were kept consistent with those in the previous period; (2) before the last ophthalmic detection, acute modeling by cold air stimulation was performed on castrated animals to accelerate modeling and facilitate observation of the drug treatment effect; and (3) after analyzing the whole experimental data, it was determined to cancel the serum sex hormone detection, tear lipid analysis and histopathology examination of lacrimal gland and meibomian gland.

### Body weight and general condition of animals

Weight change of animals: animals in the sham group kept stable weight throughout the experiment with no marked change, and animals in the Model group showed extremely significant sustained reduction of body weight throughout the experiment, indicating that animal castration procedures would significantly reduce their body weight. Animals in the EG017 low dose group, the EG017 medium dose group and the EG017 high dose group showed a rising trend in body weight during the administration period after castration, indicating that the drugs for the groups all effectively prevented the weight reduction caused by the castration of the animals.

### Effect of EG017 on tear film break-up time of modeled animals

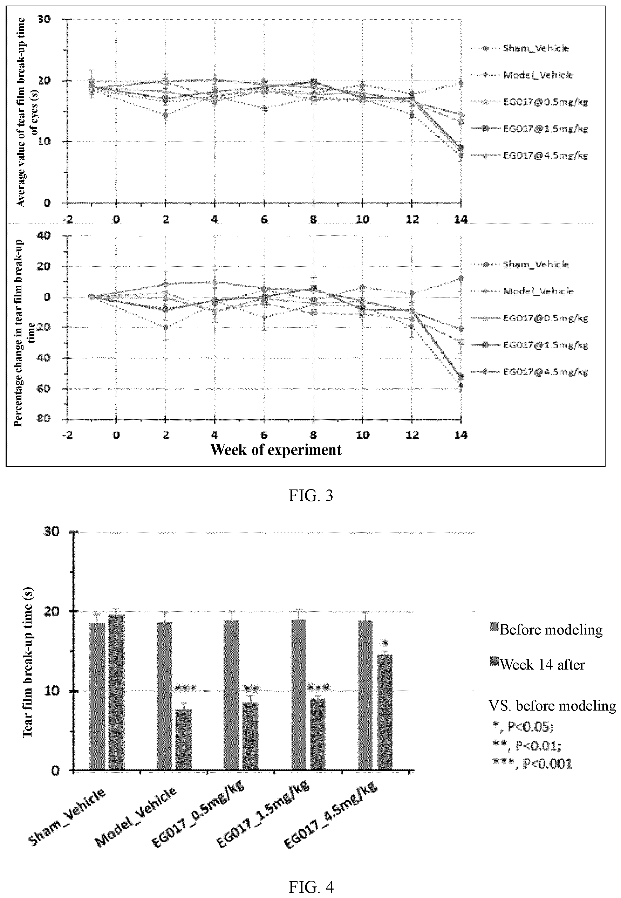

As shown in FIGs. 3 and 4, the tear film break-up time of animals in the sham group was stable and did not change markedly during the whole experiment, except for the transient decrease week 2 (P < 0.05). Animals in the Model group, the EG017 low dose group, the EG017 medium dose group and the EG017 high dose group showed no marked change with regard to the tear film break-up time within 12 weeks after modeling. The tear film break-up time of animals in the 5 groups was markedly reduced after acute modeling by cold air stimulation at week 14, indicating that the acute model of dry eye syndrome was successful. EG017 has the function of improving the tear film break-up time of animals and has dose dependence.

### Effect of EG017 on corneal fluorescence staining score for eyes

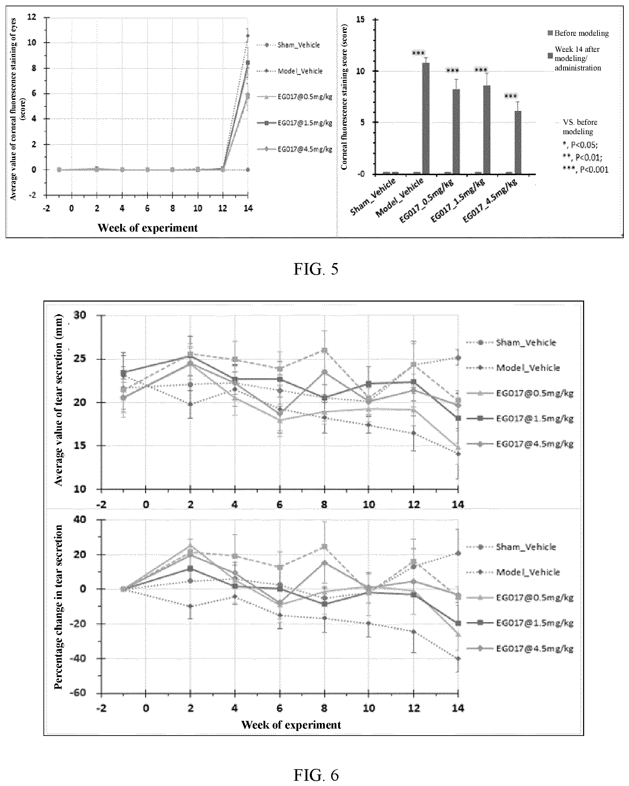

As shown in FIG. 5, the corneal fluorescence staining score for eyes of animals in all groups before modeling and at week 12 after modeling did not change markedly; when detected at week 14, after acute cold air stimulation, animals in the Model group, the EG017 low dose group, the EG017 medium dose group and the EG017 high dose group all showed score increase of different degrees, indicating that the acute modeling was successful. The Model group had a higher corneal fluorescence staining score for eyes compared with the EG017 test groups, which shows that the EG017 effectively inhibits corneal injury resulting from modeling by castration and acute modeling by cold air stimulation.

### Effect of EG017 on tear secretion of modeled animals

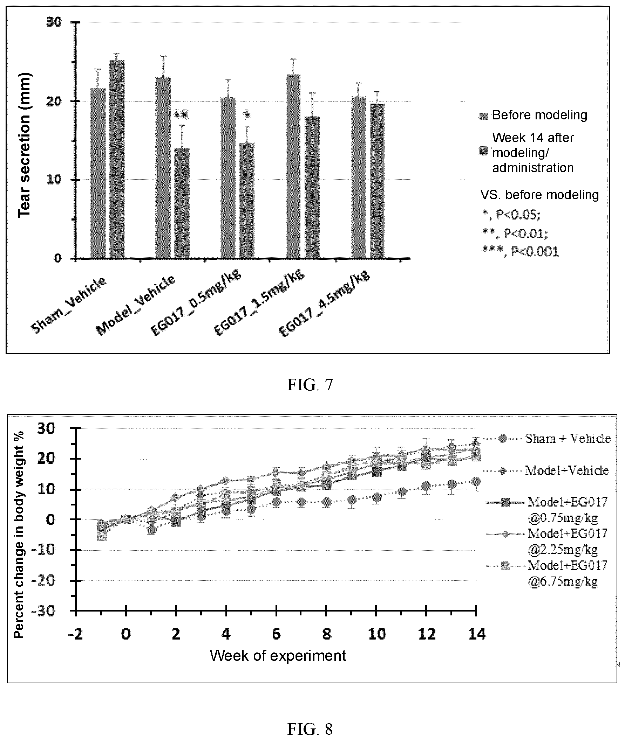

As shown in FIGs. 6 and 7, there was no marked difference in the values of tear secretion of animals in all the groups before modeling/administration, and the tear secretion of animals in the Sham group was relatively stable and showed a trend of increasing during the administration period after modeling/administration, indicating that the animals were not influenced or interfered by other factors in the whole administration period of the experiment. Animals in the Model group showed a continuous descending trend of the tear secretion index from modeling to the end point of the experiment at week 14 and had significant difference compared with other groups, indicating that the tear secretion index of the animals is significantly influenced by modeling by castration. The EG017 treatment groups could ameliorate the situation of reduction of tear secretion in animals to various degrees and showed dose correlation.

### Conclusion

Under the experimental condition, after the male cynomolgus monkey was completely castrated, its body weight was remarkably and continuously reduced, and the EG017 had good effect in inhibiting the body weight reduction and even could slightly increase the body weight. 12 weeks after castration, animals in all the groups showed no marked change in the tear film break-up time, the corneal staining and the tear secretion, except that the tear secretion of the Model group was markedly reduced from week 10 (P < 0.05), indicating that castration alone has small effect on dry eye modeling and takes a relatively long period for modeling.

To enhance the dry eye modeling effect and observe the treatment effect well, before the detection at week 14, all animals (except for the Sham group) were subjected to stimulation by continuously blowing cold air for 3 minutes at the eyes to generate an acute dry eye model; after the acute modeling, the tear film break-up time and the tear secretion of animals in the Model group were significantly reduced, and their corneal staining score was remarkably increased, indicating that the acute modeling was successful; the tear film break-up time, tear secretion and corneal staining were well ameliorated in each EG017 group and showed dose correlation.

In conclusion, EG017 has good ameliorating effect on the dry eye syndrome of the cynomolgus monkeys.

The pharmaceutical effect of the test compound EG017 (Xijian Pharmaceutical Technology Co., Ltd.) for dry eye syndrome models constructed by castration of male New Zealand white rabbits is further studied below to show its effect.

### Experimental materials

### Experimental drug and reagents

EG017: lot#: Q18-045; supplier: 2Y-Chem, Ltd.; physical properties: off-white powder; storage conditions: sealed and stored at room temperature.

Sodium carboxymethylcellulose (CMC-Na): lot#: C1814029; supplier: Aladdin; storage conditions: sealed, and stored in dry environment and at room temperature.

Xylazine hydrochloride injection (Lumianning): lot#: 170413; supplier: Jilin Huamu Animal Healthcare Products Co., Ltd.; storage conditions: sealed, and stored in dry environment and at room temperature.

0.9% sodium chloride injection: lot#: 1809271374; supplier: Cisen Pharmaceutical Co., Ltd.; storage conditions: sealed and stored at room temperature.

Fluorescein sodium: lot#: BCBQ5572V; supplier: Sigma-Aldrich (Shanghai) Trading Co., Ltd.; physical properties: orange-red powder; storage conditions: sealed and stored in dry environment.

Flunixin meglumine injection: lot#: 20180510; supplier: Jiangxi Bolai Pharmaceutical Factory Co., Ltd.

Baytril injection; lot#: 1332; manufacturer: Bayer (China) Co. Ltd.; storage conditions: stored at 2.8 °C.

Test paper strip for tear detection: lot#: 20180503 13; supplier: Tianjin Jingming New Technological Development Co., Ltd.; storage conditions: sealed and stored at room temperature.

### Main instruments:

| Instruments | Model | Manufacturer |
|---|---|---|
| Slit lamp | KOWA PRODUCT SL-17 | KOWA |
| Precision electronic balance | EX224ZH | Ohaus Instrument (Changzhou) Co., Ltd. |
| Electronic scale | ACS-30Ab | Shanghai Dahua Electronic Scale Factory |
| High-speed refrigerated centrifuge | ST 16R | ThermoFisher |
| Stopwatch | PC2810 | Shenzhen Huibo Industrial & Trading Co. Ltd. |
| Timer | WB388 | Zhanke Electronics (Shenzhen) Co., Ltd. |

### Experimental animals

36 male New Zealand white rabbits (provided by Qingdao Kangda Biotechnology Co., Ltd.), normal grade, weighed 3-4.5 kg, animal certificate No.: 37003900001046.

All the New Zealand white rabbits were fed with normal feed in an environment featuring 12-hour light/dark cycle, temperature controlled at a constant temperature (22±4 °C) and 40%-70% humidity, air change was performed 10 to 20 times per hour in the feeding room, the body weight was recorded once per week, and the handling of the animals during the experiment was in accordance with the requirements of animal ethics.

### Experimental method

### Grouping and administration for experiment

Grouping: the 36 animals were randomly divided into 6 groups based on the tear film break-up time (BUT), corneal fluorescence staining score and tear secretion (Schirmer I) before modeling, namely the sham vehicle control group (Sham), model vehicle control group (Model), EG017 0.75mg/kg low dose group (L), EG017 2.25 mg/kg medium dose group (M) and EG017 6.75 mg/kg high dose group (H), 6 animals for each group.

Administration: animals were administered with test drug as per the table below. The sham vehicle control group (Sham) and the model group (Model) were subjected to intragastric administration of 0.5% CMC-Na solvent, and the EG017 low dose, medium dose and high dose groups were subjected to intragastric administration of EG017 solution (0.75 mg/kg, 2.25 mg/kg and 6.75 mg/kg, respectively). All groups were subjected to administration for 14 consecutive weeks at a volume of 5 mL/kg once daily.

For each week, the body weight of animals was measured once and clinical observation was performed twice.

**Table 7: Administration for each group**

| **Group** | **Number** | **Operation model** | **Test drug** | **Administration dose (mg/kg)** | **Administration volume (mL/kg)** | **Route of administration** | **Frequency and period of administration** |
|---|---|---|---|---|---|---|---|
| Sham | 6 | Sham | 0.5%CMC-Na | 0 | 5 | Oral administration | Once daily, 14 weeks |
| Model | 6 | Castration | 0.5%CMC-Na | 0 | 5 | Oral administration | Once daily, 14 weeks |
| EG017 low dose | 6 | Castration | EG017 | 0.75 | 5 | Oral administration | Once daily, 14 weeks |
| EG017 medium dose | 6 | Castration | EG017 | 2.25 | 5 | Oral administration | Once daily, 14 weeks |
| EG017 high dose | 6 | Castration | EG017 | 6.75 | 5 | Oral administration | Once daily, 14 weeks |

### Establishment of animal models

Animals in the experimental groups were subjected to modeling by castration. Major steps were as follows: the animals were each subjected to general anesthesia by intramuscular injection of Lumianning (2.5 mg/kg, IM) and sterilized conventionally, and an aseptic towel was spread out. Testicles at two sides were sequentially squeezed into the scrotum from abdominal cavity and clenched to prevent them from sliding, a small opening was cut on the scrotum with a sterilized blade, the testicles were squeezed out, the spermatic vein and vas deferens were ligated, the testicles and epididymis were cut out, the scrotum skin was sutured, and iodine tincture was coated locally. After the operation, antibiotics and analgesics were injected for 1 week for nursing of the animals.

Acute modeling by cold air stimulation: because the modeling by castration operation took a relatively long time, in order to enhance the model effect, before the indexes of animals in each group (except for the Sham group) were detected for the last time, the upper eyelid and the lower eyelid of the animal were fixed to expose the eyeballs, and a high-power electric blower was used to blow cold air at the eyes of the animal from a position 10 cm away from the eyes to contiguously stimulate the eyes for 3 minutes.

### Determination of tear film break-up time (BUT)

Tear film break-up time was measured once before modeling/before administration, and was measured once every 2 weeks after administration, once for each of the left and right eyes.

Measuring method: after general anesthesia by intramuscular injection of Lumianning (2.5 mg/kg, IM), 100 µL of 0.5% fluorescein sodium was dripped into each eye without subsequent washing, and after blinking for 2-3 times, the time from opening eyes after the last blinking to the appearance of the first black spot at the cornea was observed with a slit lamp under a cobalt blue optical filter.

### Corneal fluorescence staining score

The corneal fluorescence staining score was measured once before modeling/administration, and was measured once every 2 weeks after administration, once for each of the left and right eyes.

Evaluation method: after the tear film break-up time measurement was completed, excess fluorescein solution was removed by rinsing with normal saline, and observation was performed with a slit lamp under a cobalt blue optical filter. A 12-point method was used for fluorescein staining scoring: the cornea was divided into 4 quadrants, each quadrant scoring 0-3 (scoring 0 for no staining, scoring 1 for 1-30 staining spots, scoring 2 for staining spots of greater than 30 and no fusion of staining, and scoring 3 for fusion of staining spots, filaments and ulcers at the cornea).

### Determination of tear secretion

The tear secretion was determined the day after the completion of corneal fluorescence staining under the conscious condition of the animals, and the determination was performed once before modeling/administration and once every 2 weeks after administration, once for each of the left and right eyes. A commercial test paper for tear detection was reversely folded and placed in the eyelid bitamporal conjunctival sac, the eyes of the animal were closed, and the tear infiltration length of the test paper after 5 min was detected and recorded.

### Preparation of formulations and administration

0.5% fluorescein sodium: fluorescein sodium powder was precisely weighed out and dissolved in 0.9% sodium chloride injection, thus preparing the 0.5% fluorescein sodium solution.

Preparation of 0.5% CMC-Na vehicle: CMC-Na powder was weighed out and dissolved in distilled water to prepare a CMC-Na clear solution with a final concentration of 0.5% for later use.

Preparation of EG017 solution: The administration doses for the animals were 0.75 mg/kg, 2.25 mg/kg and 6.75 mg/kg, and the administration volume was 5 mL/kg. According to the body weight of animals in each group, a proper amount of EG017 powder was weighed out one day before administration and placed in a mortar; a small amount of vehicle was added, and the mixture was fully ground; the drug liquid obtained by grinding was transferred into a graduated vessel, and the mortar was repeatedly washed with a proper amount of vehicle until no drug powder adhered to the mortar; a proper amount of vehicle was added into the graduated vessel containing the drug liquid until the administration concentration was reached, and the mixture was continuously stirred by using a magnetic stirrer until the drug liquid was completely mixed to form a suspension; the suspension was stored at 2-8 °C overnight, and administering in the morning of the next day; the drug liquid was transferred to an environment at room temperature at least 30 minutes earlier and continuously stirred with a magnetic stirrer until the administration was completed.

The EG017 solution was prepared the day before the experiment and stored in a refrigerator at 4 °C overnight after being prepared. The solution was warmed to room temperature before administration to the experimental animals, and the suspension was stirred with a magnetic stirrer during the administration period, so that the suspension was always in a stirring state, and the drug was fully suspended.

### Data processing

All data were expressed as Mean±SEM. For the experimental data, the GraphPad Prism 5 two-way ANOVA and T-test were used to analyze and compare the data between the groups and inside the group. For the statistical analysis results, P < 0.05 indicates that the difference was statistically significant, P < 0.01 indicates significant difference, and P < 0.001 indicates extremely significant difference.

### Experimental results

### Body weight and general condition of animals

The change in the body weight of animals is shown in FIG. 8. The animals in all the groups showed significant weight increase throughout the experiment, and relative to the sham group, the increase in the other groups was more significant, indicating that castration resulted in more marked weight increase of rabbits.

### Effect of EG017 on tear film break-up time

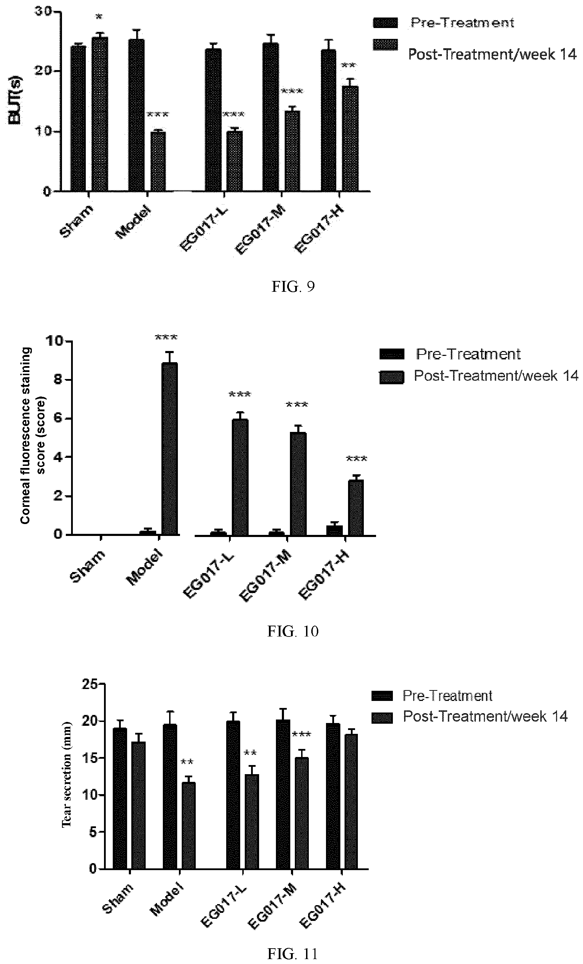

Values of tear film break-up time are shown in FIG. 9. The tear film break-up time of animals in the sham group was stable and did not change markedly during the whole experiment. Animals in the Model group, the EG017 low dose group, the EG017 medium dose group and the EG017 high dose group showed no marked change with regard to the tear film break-up time within 12 weeks after modeling. The tear film break-up time of animals in the 5 groups was markedly reduced after acute modeling by cold air stimulation at week 14 week, indicating that the acute model of dry eye syndrome was successful. EG017 has the function of improving the tear film break-up time of animals and has dose dependence.

### Effect of EG017 on corneal fluorescence staining score

The corneal fluorescence staining scores are shown in FIG. 10. The corneal fluorescence staining score for eyes of animals in all groups before modeling and at week 12 after modeling did not change markedly; when detected at week 14, after acute cold air stimulation, animals in the Model group, the EG017 low dose group, the EG017 medium dose group and the EG017 high dose group all showed score increase of different degrees, indicating that the acute modeling was successful. The Model group had a higher corneal fluorescence staining score for eyes compared with the EG017 test groups, which shows that the EG017 effectively inhibits corneal injury resulting from modeling by castration and acute modeling by cold air stimulation.

### Effect of EG017 on tear secretion

The results of tear secretion are shown in FIG. 11. There was no marked difference in the values of tear secretion of animals in all the groups before modeling and administration, and the tear secretion of animals in the Sham group was relatively stable during the administration period after modeling/administration (fluctuating increase was observed for the left eye at week 8). After acute modeling at week 14, the tear secretion of the Model group and the EG017 groups was reduced. Relative to the Model group, the EG017 treatment groups could ameliorate the situation of reduction of tear secretion in animals to various degrees and showed dose correlation.

### Conclusion and discussion

This project is intended to study the pharmacodynamics of the compound EG017 (Xijian) on the dry eye syndrome models constructed by castration of the New Zealand male rabbits. All the animals were subjected to intragastric administration the next day after modeling by castration, and the body weight of animals in the castration groups was increased more markedly than the Sham group 3 weeks after castration; 12 weeks after castration, the tear film break-up time, corneal staining and tear secretion of animals in each group showed no marked change, indicating that castration alone has small effect on dry eye modeling and takes a relatively long period for modeling.

To enhance the dry eye modeling effect and observe the treatment effect well, before the detection at week 14, all animals (except for the Sham group) were subjected to stimulation by continuously blowing cold air for 3 minutes at the eyes to generate an acute dry eye model. After the acute modeling, the tear film break-up time and the tear secretion of animals in the Model group were significantly reduced, and their corneal staining score was remarkably increased, indicating that the acute modeling was successful.

The tear film break-up time, tear secretion and corneal staining were well improved in each EG017 group and showed dose correlation.

In conclusion, the EG017 has marked ameliorating effect on the rabbit dry eye syndrome models constructed by castration and cold air blowing.

## Claims

1. Use of an ester group-containing aromatic propionamide compound for the manufacturing of a medicament for the treatment of dry eye syndrome.

2. The use of claim 1, wherein the dry eye syndrome is one caused by sex hormone imbalance.

3. The use of claim 2, wherein the ester group-containing aromatic propionamide compound is used in combination with a pharmaceutically acceptable excipient for the manufacturing of the medicament for the treatment of dry eye syndrome.

4. The use of claim 3, wherein the ester group-containing aromatic propionamide compound and the pharmaceutically acceptable excipient are manufactured into a salve.

5. The use of claim 4, wherein the pharmaceutically acceptable excipient is mineral oil and vaseline.

6. The use of claim 5, wherein the ester group-containing aromatic propionamide compound is C₂₂H₁₉F₃N₄O₄, C₂₂H₁₈F₄N₄O₄ or C₂₅H₁₇F₃N₄O₄.

7. The use of claim 6, wherein an effective dose of the C₂₅H₁₇F₃N₄O₄ is 0.5-6.75 mg/kg.
